# EUROPÄISCHE PATENTSCHRIFT

(11) **EP 1 129 692 B1**
(45) Veröffentlichungstag und Bekanntmachung des Hinweises auf die Patenterteilung: **02.06.2004**
(21) Anmeldenummer: 01105103.4
(22) Anmeldetag: 02.03.2001
(51) Int. Cl.: A61K 7/18, A61K 7/16, A61K 7/26

(54) **Mundspüllösung enthaltend Triclosan und Aminfluorid**
Mouth rinse comprising triclosan and aminofluoride
Bain de bouche comprenant du triclosan et du fluorure d'amine

(30) Priorität: 02.03.2000 DE 10010061
(43) Veröffentlichungstag der Anmeldung: 05.09.2001
(73) Patentinhaber: Dr. Scheller Cosmetics AG, 73054 Eislingen (DE)
(72) Erfinder: Casagranda, Brigitta, 73111 Lauterstein (DE); Scheller, Hans-Ulrich, Dr., 73054 Eislingen (DE); Scheller, Alexander, 73054 Eislingen (DE)
(74) Vertreter: Werner, Hans-Karsten, Dr.Dipl.-Chem.

(56) Entgegenhaltungen:
- EP-A- 0 278 744
- WO-A-98/22427
- WO-A-99/20227
- US-A- 4 512 968
- US-A- 5 176 900
- US-A- 5 925 335
- PATENT ABSTRACTS OF JAPAN vol. 1998, no. 11, 30. September 1998 (1998-09-30) & JP 10 167939 A (LION CORP), 23. Juni 1998 (1998-06-23)

## Beschreibung

Gegenstand der vorliegenden Erfindung ist eine Mundspüllösung enthaltend Triclosan, insbesondere eine Mundspüllösung, welche übliche Zusätze enthält wie Alkohol, Sorbitol, Detergenzien, Süßstoffe, Aromastoffe und Farben.

Triclosan (2,4,4--trichlor-2--hydroxy-diphenylether) ist praktisch wasserunlöslich, so dass die Mundspüllösung auch Alkohol enthalten muss, um eine ausreichende Menge an Triclosan zu lösen; vergleiche EP 0 579 383. Zur Verbesserung der Löslichkeit kann die Mundspüllösung auch Propylenglykol, Dipropylenglykol, Cellulosederivate, Olivenöl, Castoröl, Amylacetat, Ethylacetat, Glyzerintristearat oder Benzylbenzoat sowie Glyzerin und Sorbitol enthalten; vergleiche US-Patent 5,037,635.

Das US-Patent 5,328,682 beschreibt ein abrasives Mundwasser, welches durch bestimmte Zusätze eine stabile Suspension bildet, aus der sich der Putzkörper nicht absetzt. Diese Zubereitung kann als Antiplaquemittel neben vielen anderen auch Triclosan enthalten. In den Beispielen verwendet wird aber wenn überhaupt nur Bromchlorofen. Weiterhin können diese Zubereitungen Fluoridquellen enthalten. Aminfluoride kommen aber für derartige Zubereitungen schon deshalb nicht in Frage, weil die zur Stabilisierung notwendigen Polysaccharide die kationischen Aminfluoride inaktivieren.

Das US-Patent 5,290,541 beschreibt eine Mikroemulsion als Vormischung, welche unter anderem auch Triclosan enthalten kann. Es fehlt aber jeglicher Hinweis auf Aminfluoride. Auch in diesen Rezepturen wären Aminfluoride unverträglich mit den anionischen Bestandteilen.

Die DE-A-37 15 366 beschreibt eine Zahncreme mit einem Gehalt an Aminfluorid, welches zur Verhinderung der Phasentrennung Polyethylenglykol enthält. Diese Zahncreme kann zwar antibakterielle Substanzen enthalten. Es fehlt aber jeglicher Hinweis auf Triclosan. In den Beispielen sind keine antibakterielle Substanzen erwähnt.

Die DE-A-197 44 745 beschreibt Derivate von Chitosan, die besser löslich sind. Es findet sich kein Hinweis auf den Zusatz dieser Derivate in Mundwässern.

Der Nachteil der bisher bekannten Mundspüllösungen mit Triclosan besteht darin, dass sie nur sehr schwach antibakteriell wirken. Sofern also eine antibakteriell wirksame Mundspüllösung gefordert wird, ist der Zusatz von Chlorhexidin erforderlich, welches meist in Form von Chlorhexidindigluconat eingesetzt wird. Weiterhin ist als antibakteriell bekannt eine Mundspüllösung, welche die Kombination von Zinnfluorid und Aminfluoriden enthält. Der Nachteil all dieser antibakteriell wirksamen Mundspüllösungen ist, dass sie bei längerer Benutzung zu einer Verfärbung der Zähne führen und darüber hinaus einen sehr unangenehmen Geschmack aufweisen. Die Chlorhexidin enthaltenen Mundspüllösungen sind darüber hinaus überwiegend als Arzneimittel eingestuft und bedürfen daher der Zulassung.

Die Erfindung hat sich die Aufgabe gestellt, eine Mundspüllösung enthaltend Triclosan zur Verfügung zu stellen, welche ohne arzneimittelrechtliche Zulassung hergestellt und vertrieben werden kann, zu keiner Verfärbung der Zähne führt und darüber hinaus geschmacklich deutlich angenehmer ist als die bisher im Handel befindlichen antibakteriell wirksamen Mundspüllösungen.

Diese Aufgabe wird gelöst durch eine Mundspüllösung, welche außer den üblichen Zusätzen wie Alkohol, Sorbitol, Detergenzien, Süßstoffen, Aromastoffen und Farben sowie 0,005 - 0,5 Gew.-% Triclosan und 0,01 - 3,0 Gew.-% Aminfluorid enthält.

Vorzugsweise enthält die Mundspüllösung 0,02 - 0,05 Gew.-% Triclösan und 0,1 - 0,5 Gew.-% Aminfluorid.

Besonders bevorzugt ist eine Mundspüllösung, die zusätzlich 0,01 - 0,4 Gew.-% Natriumfluorid enthält.

Dokument WO 99/20227 beschreibt einen Schutzlack zur Behandlung von freiliegendem Dentin, der durch Verschluss der Dentinkanülchen vor Überempfindlichkeit der Zähne schützt und polymerisierbare Harze und Füllstoffe enthält. Nach Auftrag auf die Zähne und Lichthärtung wird ein polymeres Netzwerk gebildet, welches das Dentin vor mechanischen Einwirkungen schützt. In den Beispielen wird Triclosan in Konzentrationen von mindestens 2 Gew.% eingesetzt.

Dokument WO 98/22427 geht auf die antibiotischen Eigenschaften von Aminhydrofluoriden in Mundpflegemitteln ein.

Der Abstract von Dokument JP 10167939 (siehe PATENT ABSTRACTS OF JAPAN vol. 1998, no. 11, 30. September 1998 (1998-09-30) & JP 10 167939 A (LION CORP), 23. Juni 1998 (1998-06-23)) beschreibt ein Mundpflegemittel (z.B. eine Mundspüllösung), in dem Triclosan zu 0,01-1 Gew.% mit 1-50 Gew.% Natriumbicarbonat, 0,01-1 Gew.% Natriumfluorid und 0,001-20 Gew.% einer Kaliumionenquelle kombiniert wird.

In US-Patent 5,176,900 wird Zahnstein mit einer Mundspülung behandelt, die vorzugsweise Triclosan als antibiotische Substanz enthält. Als Fluoridionenquelle werden auch Aminfluoride vorgeschlagen, bevorzugte Fluoridionenquelle ist Natriumfluorid.

In EP-A-278 744 werden zur Densibilisierung empfindlicher Zähne eine Zahnpasta verwendet, die eine Mischung aus Triclosan und Kaliumionen enthält.

Als Aminfluorid wird vorzugsweise Olaflur (Bis(hydroxyethyl)-aminopropyl-N-hydroxyethyloctadecylamindihydrofluorid) verwendet.

Es sind aber auch andere Aminfluoride brauchbar wie Cetylaminhydrofluorid (Hexadecylaminhydrofluorid), N'N'N'-Tri-(polyoxyethylen)-N-hexadecyl-propylendiamin-dihydrofluorid und Octadecenylaminhydrofluorid.

Die antibakterielle Wirkung kann verstärkt werden durch Zusatz kationischer Filmbildner wie z.B. Chitosan in Mengen zwischen 0,01 und 5,0 Gew.-%, die sich an die Zahnoberfläche anlagern und die Adhäsion schädlicher Plaquebakterien wie Streptokokken erschwert. Chitosan wird gewonnen aus den Schalen der Shrimpsart Pandalus borealis, die in großen Mengen in der Barentsee vorkommen.

Die erfindungsgemäßen Mundspüllösungen führen zu keiner Verfärbung der Zähne, sind geschmacklich gut akzeptabel und bedürfen aufgrund ihrer Zusammensetzung keiner arzneimittelrechtlichen Zulassung. Intensive Untersuchungen und Vergleichsuntersuchungen haben gezeigt, dass die erfindungsgemäße Mundspüllösung signifikant antibakteriell ist und somit ohne weiteres anstelle der bisher handelsüblichen antibakteriellen Mundspüllösungen eingesetzt werden kann.

Die antibakterielle Wirkung ist offensichtlich auf die synergistische Kombination der Inhaltsstoffe zurückzuführen, da die Einzelkomponenten Triclosan einerseits und Aminfluoride andererseits auch in höheren Konzentrationen nicht oder nur so schwach antibakteriell sind, dass mit ihnen alleine keine brauchbare antibakterielle Mundspüllösung hergestellt werden kann. Die Untersuchungen und Vergleichsuntersuchungen wurden vor allem durchgeführt mit einer Mundspüllösung folgender Zusammensetzung: Aminfluorid (Olaflur) 33% 0,5% entsprechend 125 ppm Fluor Triclosan 0,03%

Natriumfluorid 0,028% entsprechend 125 ppm Fluor

Weiterhin enthielt diese Mundspüllösung 10% Alkohol, 5 - 10 Gew.-% Xylitol, 1 - 5 Gew.-% Polyethylenglycol-40 hydriertes Castoröl, 0,1 - 1,0 Gew.-% Aroma, 0,1 Gew.-% Acesulfam K, > 0,1 Gew.-% Farbe (CI 42051) sowie Minzgeschmack. Gewünschtenfalls enthielt die Mundspüllösung noch zusätzlich 0,5 Gew.-% Panthenol und/oder 3 Gew.-% Kaliumcitrat und/oder Extrakte von Echinacea purpurea. Der pH-Wert lässt sich auf den Bereich 3,5 bis 9, vorzugsweise 5,5 bis 8 einstellen. Hierfür sind insbesondere Zitronensäure oder Milchsäure geeignet.

Verglichen wurde diese Mundspüllösung mit einem Handelsprodukt enthaltend 0,2% Chlorhexidindigluconat, 7% Alkohol, polyoxyliertes hydriertes Rizinusöl und Aromastoffe sowie mit Plazebolösungen, die entweder nur Aminfluoride oder nur Triclosan oder keine dieser Komponenten enthielten. Untersucht wurde die Plaquebildung und die Vitalität des Plaquebiofilms. Dabei wurde festgestellt, dass die Wirksamkeit der erfindungsgemäßen Mundspüllösung zwar nicht ganz an die antibakterielle Wirkung von Chlorhexidin herankommt, jedoch deutlich und sehr signifikant wirksamer ist als Vergleichslösungen. Von den Probanden wurde der Geschmack der erfindungsgemäßen Mundspüllösung wesentlich positiver beurteilt als die im Handel befindlichen antibakteriellen Mundspüllösungen. Es wurde bei keinem der Probanden auch nach längerer Anwendung eine Verfärbung der Zähne beobachtet.

## Patentansprüche

1. Mundspüllösung enthaltend Triclosan **dadurch gekennzeichnet, dass** sie neben üblichen Zusätzen wie Alkohol, Sorbitol, Detergenzien, Süßstoffen, Aromastoffen und Farben sowie 0,005 - 0,5 Gew.-% Triclosan, 0,01 - 2,0 Gew.-% Aminfluorid enthält.

2. Mundspüllösung gemäß Anspruch 1, **dadurch gekennzeichnet, dass** sie 0,02 - 0,05 Gew.-% Triclosan und 0,1 - 0,5 Gew.-% Aminfluorid enthält.

3. Mundspüllösung gemäß Anspruch 1 oder 2, **dadurch gekennzeichnet, dass** sie zusätzlich 0,01 - 0,4 Gew.-% Natriumfluorid enthält.

4. Mundspüllösung gemäß einem der Ansprüche 1 bis 3, **dadurch gekennzeichnet, dass** das Aminfluorid Olaflur ist.

5. Mundspüllösung gemäß einem der Ansprüche 1 bis 4, **dadurch gekennzeichnet, dass** sie zusätzlich 0,01 - 5,0 Gew.-% Chitosan enthält.

6. Mundspüllösung gemäß einem der Ansprüche 1 bis 5, **dadurch gekennzeichnet, dass** sie zusätzlich bis zu 5 Gew.-% Kaliumcitrat und/oder 0,5 Gew.-% Extrakt aus Echinacea purpurea enthält.

## Claims

1. A mouth-wash solution containing triclosan, **characterized in that** it contains from 0.01 to 2.0 % by weight of amine fluoride in addition to conventional additives such as alcohol, sorbitol, detergents, sweeteners, flavouring substances and colorants and from 0.005 to 0.5 % by weight of triclosan.

2. The mouth-wash solution according to claim 1, **characterized in that** it contains from 0.02 to 0.05 % by weight of triclosan and from 0.1 to 0.5 % by weight of amine fluoride.

3. The mouth-wash solution according to claim 1 or 2, **characterized in that** it additionally contains from 0.01 to 0.4 % by weight of sodium fluoride.

4. The mouth-wash solution according to any one of claims 1 to 3, **characterized in that** the amine fluoride is olaflur.

5. The mouth-wash solution according to any one of claims 1 to 4, **characterized in that** it additionally contains from 0.01 to 5.0 % by weight of chitosan.

6. The mouth-wash solution according to any one of claims 1 to 5, **characterized in that** it additionally contains up to 5 % by weight of potassium citrate and/or 0.5 % by weight of extract from Echinacea purpurea.

## Revendications

1. Solution de bain de bouche contenant du triclosane, **caractérisée en ce qu'**elle contient, outre des adjuvants habituels comme de l'alcool, du sorbitol, des détergents, des édulcorants, des aromatisants et des colorants, également 0,005 à 0,5 % en poids de triclosane, 0,01 à 2,0 % en poids d'aminofluorure.

2. Solution de bain de bouche selon la revendication 1, **caractérisée en ce qu'**elle contient 0,02 à 0,05 % en poids de triclosane et 0,1 à 0,5 % en poids d'aminofluorure.

3. Solution de bain de bouche selon la revendication 1 ou 2, **caractérisée en ce qu'**elle contient en plus 0,01 à 0,4 % en poids de fluorure de sodium.

4. Solution de bain de bouche selon une des revendications 1 à 3, **caractérisée en ce que** l'aminofluorure est de l'Olaflur.

5. Solution de bain de bouche selon une des revendications 1 à 4, **caractérisée en ce qu'**elle contient en plus 0,01 à 5,0 % en poids de chitosane.

6. Solution de bain de bouche selon une des revendications 1 à 5, **caractérisée en ce qu'**elle contient en plus jusqu'à 5 % en poids de citrate de potassium et/ou 0,5 % en poids d'extrait d'Echinacea purpurea.
